# EUROPÄISCHE PATENTSCHRIFT

(11) **EP 1 487 322 B1**
(45) Veröffentlichungstag und Bekanntmachung des Hinweises auf die Patenterteilung: **08.08.2007**
(21) Anmeldenummer: 03745283.6
(22) Anmeldetag: 28.03.2003
(51) Int. Cl.: A61B 3/12, A61B 5/107

(54) **VERFAHREN UND VORRICHTUNG ZUR UNTERSUCHUNG VON GEF SSEN**
METHOD AND DEVICE FOR THE EXAMINATION OF CAVITIES
PROCEDE ET DISPOSITIF POUR EXAMINER DES VAISSEAUX

(30) Priorität: 28.03.2002 DE 10214360
(43) Veröffentlichungstag der Anmeldung: 22.12.2004
(73) Patentinhaber: Heidelberg Engineering Optische Messsysteme GmbH, 69221 Dossenheim (DE)
(72) Erfinder: MICHELSON, Georg, 91083 Baiersdorf (DE)
(74) Vertreter: Schmitt, Meinrad
(86) Internationale Anmeldenummer: PCT/EP2003/003264
(87) Internationale Veröffentlichungsnummer: WO 2003/082085

(56) Entgegenhaltungen:
- FISCHER JG; MEWES H; HOPP HH; SCHUBERT R: "Analysis of pressurized resistance vessel diameter changes with a low cost digital image processing device" COMPUTER METHODS AND PROGRAMS IN BIOMEDICINE, Bd. 50, Nr. 1, Juni 1996 (1996-06), Seite 23-30 XP001162760 University of Rostock, Institute of Physiology, Rostock, Germany
- NAGEL E; VILSER W; LINDLOH C; KLEIN S: "Messung retinaler Gefässdurchmesser mittels Scanning-Laser-Opthalmoskop und Computer" OPTHALMOLOGE, Bd. 89, Nr. 5, Oktober 1992 (1992-10), Seiten 432-436, XP008020465 Germany

## Beschreibung

Die Erfindung bezieht sich auf ein Verfahren zur Untersuchung von Gefäßen, insbesondere der Netzhaut, gemäß der im Oberbegriff des Patentanspruchs 1 angegebenen Merkmale. Ferner bezieht sich die Erfindung auf eine Vorrichtung zur Durchführung des Verfahrens.

Aus der EP 0 631 757 B1 sind ein derartiges Verfahren und eine derartige Vorrichtung bekannt, welche zur Messung der Fließgeschwindigkeit einer Flüssigkeit, insbesondere des Blutes, ausgebildet sind. Hierbei erfolgt die Bestimmung einer Frequenzverschiebung eines in der Flüssigkeit reflektierten Laserstrahls gemäß des optischen Doppler-Effekts, wobei mittels des Laserstrahls eine zweidimensionale, rasterförmige Abtastung durchgeführt wird. Aus der zeitlichen Variation der gemessenen Intensität des reflektierten Lichts wird die Doppler-Verschiebung berechnet und hieraus die Fließgeschwindigkeit bestimmt.

Retinale Gefäße sind der Spiegel des Gefäßsystems. Arteriosklerotische Gefäßver-änderungen im Gehirn sind assoziiert mit Veränderungen der Augenhintergrundgefäße. Generalisierte und fokale Einengungen der Netzhautgefäße, arterio-venöse Kreuzungszeichen und retinale Mikroinfarkte zeichen eine starke Korrelation mit Bluthochdruck und Schlaganfall. In einer epidemiologischen Studie (Beaver Dam Eye Study) konnte gezeigt werden, dass die Prävalenz von retinalen Emboli im Alter zwischen 43-54 Jahren 0,3%, zwischen 55-64 Jahren 1,2%, zwischen 65-74 Jahren 1,3% und bei Personen älter als 75 Jahren 3,1% beträgt. Personen mit retinalen Emboli nach Angleichung hinsichtlich Alter, Geschlecht und systematischen Faktoren zur Kontrollgruppe weisen ein 2,6-fach erhöhtes Risiko auf, infolge eines Schlaganfalles zu sterben. Die Autoren schlußfolgerten, dass das Auffinden von retinalen Emboli in einem unsymtomatischen Patienten mit Einleitung einer Frühtherapie hinsichtlich der vorliegenden Grunderkrankungen vorteilhaft für die Prognose des Patienten ist.

In einer Studie mit 11.000 Personen wurde der Augenhintergrund untersucht hinsichtlich retinaler vaskulärer Abnormalitäten und einem möglichen Zusammenhang zum arteriellen Blutdruck. Die Autoren konnten mittels konventioneller, ophtalmologischer Untersuchungsverfahren zeigen, dass mit jedem 10mmHg-Anstieg im mittleren arteriellen Druck erstens das Verhältnis aus arteriellem und venösem Durchmesser um 0,2 Einheiten sich verminderte und zweitens fokale Arterienverengungen mit einem Odds-ratio von 2 zunahmen. Weiterhin wurde eine lineare Korrelation zwischen der Höhe des Bluthochdruckes und dem retinalen Gefäßrisiko-Index ermittelt. Die dabei verarbeiteten Bilddaten wurden durch rein manuelles Ausmessen ausgewertet.

Der Schlaganfall als eine der wichtigsten Folgen der arteriellen Hypertonie ist dabei eine vermeidbare Erkrankung. Internationale Studien belegen deutlich, dass durch eine gezielte und nachhaltige Verminderung der Schlaganfall-Risikofaktoren, insbesondere des Bluthochdruckes, eine Senkung der Schlaganfall-Erkrankung um bis zu 70% erreicht werden. Entscheidend für eine erfolgreiche Prävention von Endorganschäden der arteriellen Hypertonie innerhalb der Gesamtbevölkerung ist die Erfassung von gefährdeten Patienten mit hohem vaskulären Risiko und deren Behandlung. Die Untersuchung eines retinalen Gefäß-Risiko-Indexes, der vaskuläre Schädigungen des zerebrovaskulären Gefäßsystems anzeigt, könnte als gezieltes Screening auf das Schlaganfallrisiko bei großen Teilen der Bevölkerung eingesetzt werden. Bei vielen vaskulären Erkrankungen liegt die Ursache der Sauerstoffmangelversorgung in einer Verdickung der Gefäßwand. Es ist bekannt, dass die Dicke der Intima-Media der A.carotis interna einen sehr wichtigen Risikofaktor bei der Beurteilung des Arteriosklerose-Risikos darstellt.

Hiervon ausgehend liegt der Erfindung die Aufgabe zugrunde, das Verfahren und die Vorrichtung der genannten Art dahingehend weiterzubilden, dass mit einem geringen Aufwand weitere Untersuchungen von Netzhautgefäßen durchführen zu können. Die Untersuchungen sollen genaue Meßergebnisse liefern, welche insbesondere Aufschluß über etwaige Erkrankungen bzw. über den Gesundheitszustand der untersuchten Person geben sollen. Des Weiteren sollen neue Anwendungs- und Einsatzgebiete des Verfahrens und der Vorrichtung, welche auch als Ophtalmoskop bezeichnet wird, erschließen.

Die Lösung dieser Aufgabe erfolgt hinsichtlich des Verfahrens gemäß der im Patentanspruch 1 angegeben Merkmale. Hinsichtlich des Verfahrens erfolgt die Lösung gemäß der im Patentanspruch 5 angegebenen Merkmale.

Das erfindungsgemäß vorgeschlagene Verfahren und die zur Durchführung desselben vorgeschlagene Vorrichtung ermöglichen ohne besonderen Aufwand die Ermittlung der Gefäßwanddicke retinaler Gefäße. Hierdurch wird eine erheblich erweiterte Einsatzmöglichkeit von abtastenden Lasergeräten und/oder Ophtalmoskopen geschaffen. Insbesondere die eingangs erwähnten Untersuchungen können mit hoher Präzision durchgeführt werden, damit aufgrund somit festgestellter Risiken die notwendigen Therapien eingeleitet werden können. Die erfindungsgemäß nicht-invasive Bestimmung der Gefäßwanddicke retinaler Gefäße ermöglicht mit minimalem Aufwand ein Screening breiter Bevölkerungsschichten, wobei durch Früherkennung etwaiger Erkrankungen die Lebensqualität der betroffenen Patienten wesentlich verbessert werden kann und zudem erhebliche Kostenreduzierungen im Gesundheitswesen erzielt werden können.

Die Meßmethode der Scanning Laser Doppler Flowmetrie ermöglicht die simultane Darstellung von Netzhaugefäßen mittels der Reflektivität und mittels des Laser Doppler Shifts. Die Refelktivitätsbilder liefern den äußeren Durchmesser der retinalen Gefäße, die Dopplerbilder liefern den Durchmesser der bewegten Blutsäule. Aus der Differenz der beiden Bilder wird die Gefäßwanddicke retinaler Gefäße ermittelt. Die Gefäßwanddicke kann bestimmt werden durch Vergleich der bewegten Blutsäule (Laser Doppler Bild) mit Gesamtgefäß (Reflektivitätsbild). Da die Netzhaut ein sichtbarer Teil des Gehirns darstellt, kann durch Beobachtung von Netzhautgefäßen das vaskuläre Risiko der Gehirnversorgung beurteilt werden. Ein sehr wichtiger Parameter ist die Gefäßwanddicke. Erfindungsgemäß erfolgt eine nicht-invasive Bestimmung der Gefäßwanddicke retinaler Gefäße mittels Scanning Laser Doppler.

Weiterbildungen und besondere Ausgestaltungen der Erfindung sind in den Unteransprüchen und der nachfolgenden Beschreibung angegeben.

Die Erfindung wird nachfolgend anhand der Zeichnung näher erläutert, ohne dass insoweit eine Beschränkung erfolgt.

Es zeigen:
- Fig. 1: ein Diagramm mit aus einem Reflektivitätsbild ermittelten Daten eines Netzhautgefäßes,
- Fig. 2: das der Fig. 1 zugrundeliegende Reflektivitätsbild"
- Fig. 3: ein Diagramm der aus einem Laser Doppler Bild ermittelten Daten eines Netzhautgefäßes,
- Fig. 4: das der Fig. 3 zugrundeliegende Laser Doppler Bild.

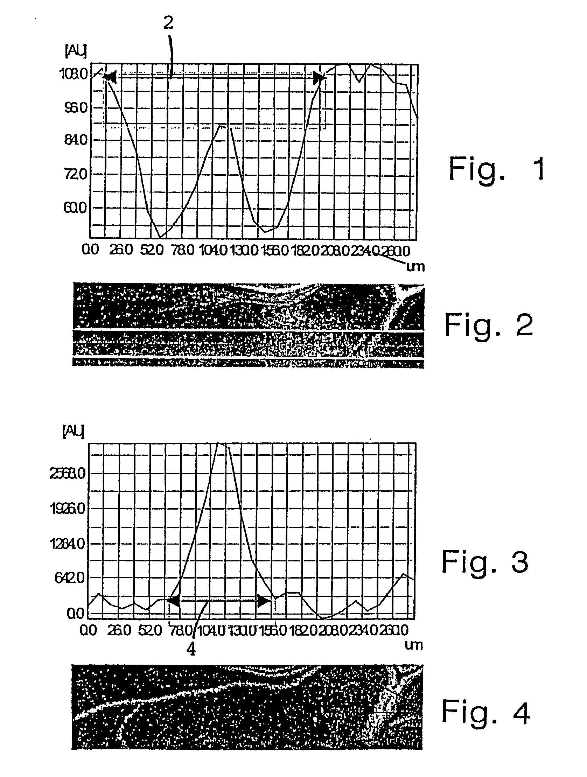

Fig. 1 zeigt ein typisches Diagramm, entsprechend eines Netzhautgefäßes, dessen Reflektivitätsbild in Fig. 2 dargestellt ist. Mit dem Pfeil 2 ist der äußere Durchmesser des Gefäßes markiert. Hierbei wird zumindest näherungsweise der Abstand der beiden maximalen Werte erfaßt, zwischen welchen die den Gefäßwänden entsprechenden Minima liegen. Die Abzissenteilung ist in Mikrometer angegeben und somit ist ein Gefäßaußendurchmesser von 190µm gegeben.

Das Diagramm gemäß Fig. 2 zeigt einen typischen Verlauf entsprechend einer bewegten Blutsäule nach Auwertung des in Fig. 3 dargestellten Laser-Doppler-Bildes. Ein derartiges Laser-Doppler-Bild kann beispielsweise durch das in der erwähnten EP 0 631 757 B1 beschriebene Verfahren zur Messung der Fließgeschwindigkeit des Blutes bzw. des entsprechend ausgebildeten Gerätes erzeugen. Der Erfinder hat erkannt, dass durch die Abbildung und Erfassung der bewegten Blutsäule auf den Innendurchmesser des Gefäßes geschlossen werden kann. Bis heute ist unter praktischen Gesichtpunkten und insbesondere einer erzielbaren Meßgenauigkeit eine direkten Ausmessung des Gefäßinneren ist nicht durchführbar. Vielmehr wird gemäß der Erfindung mittelbar über die Erfassung der bewegten Blutsäule der Innendurchmesser bestimmt. Unter Berücksichtigung des in µm angegeben Abszissenmaßstabes des Diagramms 2 gemäß Pfeil 4 weist das Gefäß einen Innendurchmesser von 90µm auf.

Aus den derart ermittelten Werten für den äußeren Durchmesser und den inneren Durchmesser des Gefäßes wird in bevorzugter Weise und mit geringem Aufwand, insbesondere an Rechnerleistung, durch Differenzbildung oder Korrelation dieser Daten die Gefäßwanddicke bestimmt.

In der Vorrichtung zur Durchführung des Verfahrens gelangt die in einem Lasergerät zur Augenhintergrundsuntersuchung bzw. Bildauswertung vorgesehene Auswerteeinheit zum Einsatz, wobei zusätzlich der Rechner der Vorrichtung dahingehend ausgebildet ist, dass die erfindungsgemäß durchzuführende Differenzbildung bzw. Korrelation der ermittelten Daten durchgeführt wird.

## Patentansprüche

1. Verfahren zur Untersuchung von Gefäßen, insbesondere eines Auges, mittels Laserabstastung ,
**dadurch gekennzeichnet, dass** die Bestimmung der Gefäßwanddicke, insbesondere retinaler Gefäße, durchgeführt wird, wobei mittels Scanning Laser Doppler zum einen der äußere Durchmesser und zum anderen der innere Durchmesser des Gefäßes bestimmt wird und dass aus den derart ermittelten Daten die Wanddicke des Gefäßes bestimmt wird.

2. Verfahren nach Anspruch 1, **dadurch gekennzeichnet, dass** der äußere Gefäßdurchmesser aus den Daten eines Reflektivitätsbildes bestimmt wird.

3. Verfahren nach Anspruch 1 oder 2, **dadurch gekennzeichnet, dass** der innere Durchmesser durch die Bestimmung des Durchmessers der bewegten Blutsäule, insbesondere aus den Daten eines Laser Doppler Bildes bestimmt wird.

4. Verfahren nach einem der Ansprüche 1 bis 3, **dadurch gekennzeichnet, dass** die Gefäßwanddicke durch Differenzbildung der aufgrund eines Reflektivitätsbildes einerseits und eines Laser Doppler Bildes andererseits erfaßten Daten bestimmt wird.

5. Vorrichtung zur Durchführung des Verfahrens nach einem der Ansprüche 1 bis 4, **dadurch gekennzeichnet, dass** ein Scanning Laser Doppler System vorgesehen ist, mittels welchem einerseits ein Reflektivitätsbild des Gefäßes, insbesondere dessen Außendurchmesser, und/oder dem Gefäßaußendurchmesser entsprechende Daten erzeugbar sind, und andererseits ein Laser Doppler Bild, insbesondere der bewegten Blutsäule und/oder des inneren Gefäßdurchmessers und/oder diesem korrespondierende Daten erzeugbar sind und dass eine Auswerteinheit vorgesehen ist zur Bestimmung der Gefäßwanddicke aus den derart ermittelten Bildern und/oder Daten.

6. Vorrichtung nach Anspruch 5, **dadurch gekennzeichnet, dass** die Auswerteinheit als ein Rechner ausgebildet ist, mittels welchem bevorzugt durch Korrelation oder Differenzbildung, aus den genannten Bildern und/oder Daten die Gefäßwanddicke bestimmbar ist.

## Claims

1. Method for examining blood vessels, in particular of an eye, by means of laser scanning, **characterised in that** the thickness of vascular walls, in particular of retinal blood vessels is determined, the outer diameter on the one hand and the inner diameter of the blood vessel on the other being determined by means of laser Doppler scanning, and **in that** the thickness of the vascular wall is determined from the data obtained in this manner.

2. Method according to claim 1, **characterised in that** the outer diameter of the blood vessel is determined from the data obtained from a reflectivity image.

3. Method according to either claim 1 or claim 2, **characterised in that** the inner diameter is determined by determining the diameter of the moving blood column, in particular from the data obtained from a laser Doppler image.

4. Method according to any one of claims 1 to 3, **characterised in that** the thickness of the vascular walls is determined by ascertaining the difference in the data obtained from the reflectivity image on the one hand and the laser Doppler image on the other.

5. Device for carrying out the method according to any one of claims 1 to 4, **characterised in that** a laser Doppler scanning system is provided, by means of which, on the one hand, a reflectivity image of the blood vessel, in particular of its outer diameter, and/or data corresponding to the outer diameter of the blood vessel may be produced and, on the other hand, a laser Doppler image, in particular of the moving blood column and/or of the inner diameter of the blood vessel and/or data corresponding thereto may be produced and **in that** an evaluation unit is provided to determine the thickness of the vascular wall from the images and/or data obtained in this manner.

6. Device according to claim 5, **characterised in that** the evaluation unit is configured as a computer, by means of which the thickness of the vascular wall may be determined. preferably by correlation or by ascertaining the difference, from the aforementioned images and/or data.

## Revendications

1. Procédé pour l'examen de vaisseaux, en particulier d'un oeil, par balayage laser,
***caractérisé en ce qu'***on procède à la détermination de l'épaisseur de la paroi de vaisseaux, en particulier des vaisseaux rétiniens, d'une part le diamètre extérieur et d'autre part le diamètre intérieur du vaisseau étant déterminés par Doppler à laser à balayage, et ***en ce que*** l'épaisseur de paroi du vaisseau est déterminée à partir des données ainsi relevées.

2. Procédé selon la revendication 1, ***caractérisé en ce que*** le diamètre extérieur du vaisseau est déterminé à partir des données d'une image de réflectivité.

3. Procédé selon la revendication 1 ou 2, ***caractérisé en ce que*** le diamètre intérieur est déterminé par détermination du diamètre de la colonne de sang en mouvement, en particulier à partir des données d'une image laser à effet Doppler.

4. Procédé selon l'une quelconque des revendications 1 à 3, ***caractérisé en ce que*** l'épaisseur de paroi du vaisseau est déterminée en calculant la différence entre des données relevées d'une part à partir d'une image de réflectivité et d'autre part à partir d'une image laser à effet Doppler.

5. Dispositif de mise en oeuvre du procédé selon l'une quelconque des revendications 1 à 4, ***caractérisé en ce qu'***un système de balayage laser à effet Doppler est prévu, au moyen duquel d'une part une image de réflectivité du vaisseau, en particulier de son diamètre extérieur, et/ou des données correspondant au diamètre extérieur du vaisseau peu(ven)t être produite(s), et d'autre part une image laser à effet Doppler, en particulier de la colonne de sang en mouvement et/ou du diamètre intérieur du vaisseau et/ou des données correspondant à celui-ci peu(ven)t être produite(s), et *en ce qu*'une unité d'analyse est prévue pour déterminer l'épaisseur de paroi du vaisseau à partir des images et/ou données ainsi relevées.

6. Dispositif selon la revendication 5, ***caractérisé en ce que*** l'unité d'analyse est réalisée sous la forme d'un ordinateur, au moyen duquel l'épaisseur de paroi du vaisseau peut être déterminée de préférence par corrélation ou différence à partir desdites images et/ou données.
